(19) Europäisches Patentamt / European Patent Office / Office européen des brevets

(11) **EP 2 193 114 B1**

(12) **FASCICULE DE BREVET EUROPEEN**

(45) Date de publication et mention
de la délivrance du brevet:
**06.03.2013 Bulletin 2013/10**

(21) Numéro de dépôt: **08805015.8**

(22) Date de dépôt: **02.10.2008**

(51) Int Cl.:
**C07C 209/48** *(2006.01)*    **C07C 211/12** *(2006.01)*

(86) Numéro de dépôt international:
**PCT/EP2008/063233**

(87) Numéro de publication internationale:
**WO 2009/043906 (09.04.2009 Gazette 2009/15)**

(54) **PROCEDE DE FABRICATION D'AMINES PAR HYDROGENATION DE COMPOSES NITRILES**

VERFAHREN ZUR HERSTELLUNG VON AMINEN DURCH HYDRIERUNG EINER
NITRILVERBINDUNG

METHOD FOR PRODUCING AMINES BY NITRILE COMPOUND HYDROGENATION

(84) Etats contractants désignés:
**AT BE BG CH CY CZ DE DK EE ES FI FR GB GR
HR HU IE IS IT LI LT LU LV MC MT NL NO PL PT
RO SE SI SK TR**

(30) Priorité: **04.10.2007  FR 0706966**

(43) Date de publication de la demande:
**09.06.2010   Bulletin 2010/23**

(73) Titulaire: **Rhodia Opérations
93306 Aubervilliers (FR)**

(72) Inventeurs:
• **LETOURNEUR, Didier
F-68170 Rixheim (FR)**
• **LECONTE, Philippe
F-68150 Ribeauvillé (FR)**
• **SPINDLER, Jean-Francis
F-69360 Ternay (FR)**
• **LERMUSIAUX, Patrick
F-69006 Lyon (FR)**
• **BOSCHAT, Vincent
F-03300 Cusset (FR)**

(74) Mandataire: **Chatelan, Florence Anne et al
Rhodia Services
Direction Propriété Industrielle
85 rue des Frères Perret
69192 Saint-Fons Cedex (FR)**

(56) Documents cités:
**WO-A-99/26917    FR-A- 2 773 086**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

La présente invention concerne un procédé de fabrication de composés amines par hydrogénation de composés nitriles en présence d'un catalyseur.

Elle concerne plus particulièrement un procédé de fabrication de diamines par hydrogénation, en continu, de composés dinitriles en présence d'un catalyseur à base d'un métal de Raney et en absence de solvant alcoolique.

Un des procédés de fabrication de composés amines utilisé industriellement est l'hydrogénation catalytique de composés nitriles.

Ainsi, l'hexaméthylène diamine qui est un intermédiaire chimique important pour la fabrication de nombreux composés ou polymères, est produite, majoritairement, par hydrogénation catalytique de l'adiponitrile.

A titre d'exemple, on peut citer les procédés d'hydrogénation de l'adiponitrile en présence d'un oxyde métallique, comme catalyseur tel que l'oxyde de fer ou de cobalt. Ces procédés sont généralement mis en oeuvre à des pressions et températures élevées et souvent en présence d'ammoniac.

D'autres procédés d'hydrogénation largement exploités consistent à utiliser comme catalyseur, un système à base d'un métal de Raney, plus particulièrement de Nickel ou Cobalt de Raney, en présence d'alcool, ou d'eau et d'un composé basique comme cocatalyseur. Dans ces procédés, la réaction est réalisée à des pressions et températures peu élevées et en absence d'ammoniac. Ces derniers procédés d'hydrogénation avec catalyseur à base de métal de Raney peuvent être mis en oeuvre dans certains types de réacteur. En effet, comme le catalyseur est pyrophorique, les réacteurs utilisés doivent permettre un contrôle efficace de l'introduction en continu du catalyseur dans le milieu réactionnel. Il est également nécessaire de contrôler la quantité de catalyseur mise en suspension dans le milieu réactionnel et d'assurer une circulation maîtrisée et contrôlée de cette suspension.

Cependant, l'activité de ces catalyseurs peut être fortement affectée quand ils sont utilisés sous certaines conditions dans les procédés d'hydrogénation de composés nitriles.
Un article publié dans Chemical Engineering Science vol 47 n°9-11, 2 289-94 (1992) indique que les composés nitriles désactivent les catalyseurs à base de Nickel ou Cobalt de Raney. Pour diminuer cette désactivation, il a été proposé, dans l'article publié dans Chemical Engineering Science Vol 35, 135-141 (1980) d'utiliser un réacteur avec une vitesse de circulation élevée du milieu réactionnel pour obtenir des conditions de mélanges optimales et un flux turbulent pour éviter la formation de zones présentant une concentration élevée en composés nitriles. En effet, une concentration élevée en composés nitriles induit une désactivation du catalyseur.

Tous ces procédés utilisent comme solution pour diminuer la dégradation de l'activité du catalyseur, des systèmes permettant d'avoir un mélange efficace du milieu réactionnel et donc avoir une concentration en réactifs et catalyseur identiques en tout point du réacteur.

Une telle qualité de mélange est difficile à obtenir et demande des dispositifs complexes comme ceux décrits dans la demande de brevet WO00/37424 qui concerne l'utilisation de mélangeurs statiques pour améliorer la qualité du mélange et l'homogénéité des concentrations en tout point du réacteur.

En outre, ces solutions peuvent s'appliquer quand la réaction d'hydrogénation est mise en oeuvre, en continu, dans un réacteur fonctionnant sous un régime piston tel que les colonnes à bulles fonctionnant avec circulation d'un lit de catalyseur en suspension, les réacteurs comprenant une colonne à bulles et une tête cyclone pour séparer les gaz, appelés ci-après réacteurs de type RTC.

Une autre caractéristique de ces procédés réside dans le phénomène de désactivation rapide du catalyseur par formation et dépôt d'aluminate sur le catalyseur.

Pour éviter ce problème ou le diminuer, il avait été proposé d'ajouter un alcool dans le milieu réactionnel pour permettre de dissoudre l'aluminate et limiter son dépôt sur le catalyseur. Toutefois, il était nécessaire de réaliser des arrêts périodiques de l'installation pour nettoyer et éliminer les dépôts d'aluminate sur les parties de l'appareillage dus à la cristallisation de l'aluminate.

Ce procédé avait également comme inconvénient de comprendre des opérations de distillation et séparation de l'alcool. En outre, l'alcool pouvait réagir avec l'amine formée telle que l'hexaméthylène diamine (HMD) pour former une impureté telle que l'imine de la N-éthyl-héxaméthylène diamine, difficilement séparable de la diamine formée.
Il a également été proposé des procédés d'hydrogénation sans alcool mais avec de l'eau tel que décrit dans les brevets US 3 056 837, 4 429 159 et 4 491 673, par exemple.

Toutefois, pour maintenir une activité élevée du catalyseur dans le milieu réactionnel, il était nécessaire de soutirer une quantité importante de catalyseur usé et de remplacer cette quantité de catalyseur par du catalyseur vierge. La consommation en catalyseur était donc élevée et affectait l'économie du procédé.

Par ailleurs, le document WO99/26917 décrit un procédé continu d'hydrogénation de dinitrile, au moins en partie en aminonitrile correspondant, en présence d'un catalyseur d'hydrogénation non-dissous dans le milieu réactionnel.

Le document FR2773086 décrit un procédé de régénération de catalyseurs de type catalyseur de Raney d'hydrogénation totale ou partielle des fonctions nitriles en fonctions amines de composés organiques, qui consiste à mélanger le catalyseur utilisé avec une solution aqueuse d'un composé basique présentant une certaine concentration en ions basiques.

Un des buts de la présente invention est de proposer un procédé continu d'hydrogénation de composés nitriles en composés amines en présence d'un catalyseur à base de métal de Raney dans un milieu comprenant de l'eau, en absence d'alcool, permettant de maintenir une activité catalytique convenable dans le réacteur avec une consommation faible de catalyseur par tonne de composé amine produite.

A cet effet, l'invention propose un procédé de fabrication de composés comprenant au moins une fonction amine par hydrogénation d'un composé comprenant au moins une fonction nitrile consistant à :

- alimenter un gaz contenant de l'hydrogène et un composant comprenant au moins une fonction nitrile, dans un réacteur de type piston dans lequel circule un milieu réactionnel comprenant des particules en suspension de catalyseur à base de métal de Raney, une base minérale et de l'eau,
- soutirer en sortie du réacteur piston, une partie du milieu réactionnel contenant le composé comprenant au moins une fonction amine après séparation du catalyseur et recycler l'autre partie dans le réacteur piston
- recycler le catalyseur séparé dans le réacteur piston
- alimenter dans le réacteur piston un flux de catalyseur vierge,

caractérisé en ce qu'une partie du catalyseur séparé est soumis à un procédé de régénération avant d'être recyclé dans le milieu réactionnel, ledit procédé de régénération comprenant une étape de lavage à l'eau du catalyseur pour éliminer la plus grande partie des composés organiques, le

catalyseur lavé étant soumis à un traitement avec une base pour dissoudre la plus grande partie des aluminates formés suivi d'un lavage avec une solution aqueuse d'hydroxyde alcalin et/ou de l'eau.

Par catalyseur vierge, on entend un catalyseur n'ayant pas été utilisé dans une réaction d'hydrogénation. Selon une caractéristique de l'invention, le débit massique R de catalyseur régénéré recyclé et le débit N massique de catalyseur vierge alimenté doit avantageusement satisfaire à l'équation I suivante :

$$0,60 \leq \frac{R}{R + N} \leq 0,95 \quad (I)$$

De préférence, ces débits R et N doivent avantageusement satisfaire à l'équation II suivante :

$$0,70 \leq \frac{R}{R + N} \leq 0,90 \quad (II)$$

Ce contrôle des débits, R et N de catalyseurs régénéré et vierge permet de contrôler l'activité moyenne du catalyseur dans le milieu réactionnel et ainsi optimiser le débit de catalyseur vierge pour minimiser la consommation de catalyseur par tonne d'amine produite tout en maintenant des conditions optimales de fonctionnement du procédé, par exemple, taux de transformation du nitrile, sélectivité en diamine.

Les débits massiques R et N sont exprimés en poids de catalyseur par unité de temps. Le débit moyen massique de catalyseur vierge N est déterminé par la productivité souhaitée de l'installation ou du procédé d'hydrogénation et plus particulièrement dépend du débit de composés nitriles alimentés dans l'installation.

Les catalyseurs convenables pour l'invention sont les métaux de Raney tels que le nickel de Raney ou le cobalt de Raney, préférentiellement le nickel de Raney.

Des éléments promoteurs peuvent être avantageusement utilisés avec le métal de Raney. Ces éléments promoteurs sont choisis dans les éléments appartenant aux groupes IIB, IVB à VIIB de la classification périodique des éléments. Avantageusement, les éléments promoteurs sont choisis dans le groupe comprenant le titane, le chrome, le zirconium, le vanadium, le molybdène, le manganèse, le cobalt, le nickel, le zinc, le fer et leurs associations.

Selon le procédé de l'invention, la partie de catalyseur séparé du milieu réactionnel est soumise à un procédé de régénération permettant d'obtenir une activité catalytique correspondant de 35% à 80%, de préférence de 40% à 70% de l'activité catalytique d'un catalyseur vierge.

Cette activité du catalyseur est exprimée par la quantité molaire d'hydrogène consommée lors de la réduction de l'adiponitrile en hexaméthylène diamine pour une quantité de catalyseur Nickel de Raney définie par unité de temps dans des conditions de température et de pression et un rapport KOH/masse de catalyseur déterminés pour la méthode de mesure.

L'activité initiale du catalyseur est déterminée en mettant en oeuvre le mode opératoire décrit ci-dessous :

Dans un bécher de 250 ml, verser 50 ml d'eau déminéralisée et désaérée à l'argon.

A l'aide d'une spatule, prélever environ 2 grammes de bouillie de catalyseur à analyser

Introduire la prise d'essai dans le bécher et ajouter environ 100ml d'eau déminéralisée et désaérée.

Agiter le milieu pour mettre en suspension le catalyseur à analyser.

Séparer l'eau du catalyseur en posant une plaque aimantée au fond du bécher.

Vider l'eau déminéralisée et désaérée.

Verser 150 ml d'eau déminéralisée et désaérée dans le bécher.

Le bécher posé sur l'aimant : vider l'eau de lavage puis ajouter 50 à 100ml d'eau déminéralisée et désaérée.

Laver le catalyseur à l'eau déminéralisée et désaérée. A la fin des lavages, le pH de l'eau doit être voisin de 7.

Remplir un pycnomètre avec le liquide surnageant du catalyseur.

Peser le pycnomètre pour définir le zéro de la balance.

Enlever le pycnomètre de la balance.

Enlever une petite quantité d'eau du picnomètre avec une pipette.

Prélever dans le bécher, le catalyseur avec la même pipette.

Introduire une quantité de catalyseur dans le picnomètre et réajuster le pycnomètre avec le liquide surnageant.

Peser le pycnomètre et lire la différence de masse (M) entre le pycnomètre rempli d'eau plus le catalyseur et le pycnomètre rempli d'eau. La masse de catalyseur $m_{catalyseur}$ est égale à 1,15*M. Réajuster la quantité de catalyseur dans le pycnomètre en répétant les opérations de pesée ci-dessus jusqu'à obtenir la quantité désirée :

La quantité de catalyseur à prélever est avantageusement égale à :

- 0,4000 g environ exactement de catalyseur lavé neuf, régénéré ou usé :

$$(0,3960 \text{ g} \leq m_{catalyseur} \leq 0,4040 \text{ g})$$

Noter la masse $m_{catalyseur}$ de catalyseur introduite dans le pycnomètre (précision :0,0001 gramme). Poser le réacteur propre et sec, ainsi que son support sur le trébuchet. Faire le zéro.

Transvaser le contenu du pycnomètre dans un réacteur propre et sec.

Rincer le pycnomètre avec la quantité nécessaire d'eau déminéralisée pour recueillir la totalité du catalyseur introduit dans le pycnomètre.

Eliminer le maximum d'eau en utilisant la plaque aimantée sur le fond du réacteur.

Ajouter sur le catalyseur de la potasse KOH 6.8N à l'aide de la micro seringue adaptée soit 47 $\mu$l

Compléter avec de l'eau déminéralisée pour obtenir un poids de solution dans le réacteur égal à 4,66 g

Ajouter 37,8 g d'HMD pure dans le réacteur.

Installer le réacteur sur le dispositif pilote et mettre le chauffage en route (Température de consigne 80°C).

Purger 3 fois le ciel du réacteur avec de l'azote.

Mettre la réserve d'hydrogène à une pression de 50 bar.

Purger le ciel du réacteur à l'hydrogène par admission d'hydrogène et évacuation. Répéter l'opération 3 fois en maintenant ou rétablissant la pression dans la réserve d'hydrogène.

A la fin des trois purges à l'hydrogène du réacteur, pressuriser le réacteur à 20 bar en hydrogène.

Mettre l'agitation en route (température de consigne de 80°C et vitesse d'agitation à 2000 trs.min-1).

Prélever environ exactement 6,00 grammes d'adiponitrile avec une seringue en verre munie de son aiguille rallongée.

Faire le zéro de la balance avec la seringue chargée et munie de son aiguille.

Introduire l'adiponitrile dans une ampoule de coulée.

Peser la seringue vide munie de son aiguille.

Noter la masse d'adiponitrile $m_{ADN}$ introduite dans l'ampoule de coulée (elle doit être comprise entre 5,70 et 6,30 grammes).

Mettre le réacteur sous pression d'hydrogène à partir de la réserve d'hydrogène et rétablir une pression de 50 bar dans la réserve. Vérifier que les pressions de réserve et de réacteur restent constantes.

Une fois la température et l'agitation atteintes, ouvrir la vanne de l'ampoule de coulée d'abord doucement puis complètement en une seule opération pour introduire l'AdN dans le réacteur.

La pression à l'intérieur du réacteur doit monter à 25 bar.

L'alimentation en hydrogène dans le réacteur à partir de la réserve est maintenue ouverte jusqu'à ce que la courbe de pression d'hydrogène soit stable.

Pendant la durée de la réaction, la pression d'hydrogène dans la réserve est enregistrée en fonction du temps.
Quand la pression d'hydrogène dans le réacteur est stable, l'alimentation en hydrogène dans le réacteur est fermée.
Le chauffage du réacteur est coupé et le milieu réactionnel est refroidi jusqu'à une température de 45°C.
Décomprimer doucement le réacteur et arrêter l'agitation.
Purger trois fois le ciel du réacteur avec de l'azote.
Vider le contenu du réacteur dans un récipient avec rinçage à l'eau déminéralisée.
L'activité du catalyseur est déterminée par la vitesse initiale de la réaction donnée par la formule suivante :

$$V_{initiale} = \frac{4 \ x \ m_{ADN}}{108 \ x \ m_{catalyseur} \ x \ 60 \ x \ \Delta t_{initial}}$$

$\Delta t_{initial}$ est le temps en minute que mettrait l'hydrogénation si le catalyseur était renouvelé en permanence durant la réaction. Il est déterminé par l'abscisse du point d'intersection entre la pente de la courbe d'enregistrement de la pression au départ de la réaction et la pente à la fin de la réaction qui est généralement nulle. Cette courbe représente la variation de la pression d'hydrogène dans la réserve en fonction du temps.
La vitesse initiale d'hydrogénation est exprimée en mole $H_2$. $g^{-1}cata.s^{-1}$.
Selon l'invention le procédé de régénération comprend une première étape de lavage du catalyseur à régénérer pour éliminer la plus grande partie des composés organiques, notamment de l'amine formée telle que l'hexaméthylène diamine, par exemple.
Ce lavage est effectué avec de l'eau en réalisant avantageusement plusieurs lavages successifs pour obtenir dans la dernière eau de lavage une concentration pondérale en composés organiques inférieure ou égale à 1 % en poids. De préférence, cette concentration en composés organiques est mesurée par détermination de la quantité d'amine contenue dans l'eau de lavage.
Ce lavage peut également être réalisé en continu dans une ou plusieurs colonnes à flux à contre-courant ou dans une colonne comprenant le catalyseur à laver sous forme de lit-fixe.
Avantageusement, le lavage est réalisé avec de l'eau à une température comprise entre 10°C et 50°C, préférentiellement avec de l'eau à température ambiante (20-25°C), pour éviter l'encrassement des colonnes ou laveurs.
Le catalyseur lavé est ensuite soumis à un traitement par une base forte pour notamment dissoudre les aluminates formés. Comme base convenable, on peut utiliser de préférence les hydroxydes alcalins, la soude étant l'hydroxyde préféré.
La solution de base utilisée contient de 10% à 25% en poids de soude.
Le traitement est de préférence, réalisé à une température supérieure à 80°C et avantageusement à la température d'ébullition de la solution de soude.
Il peut être réalisé dans un procédé discontinu ou dans un procédé continu.
La durée de traitement par la soude doit être suffisante pour permettre une régénération de l'activité catalytique correspondant à au moins 35% de l'activité catalytique d'un catalyseur vierge, avantageusement au moins 39 %.
En outre, le traitement avec un composé basique permet de produire de l'hydrogène par attaque de l'aluminium métal.
Cet hydrogène permet de conditionner le catalyseur.
Le catalyseur ainsi traité est soumis à une nouvelle étape de lavage pour éliminer les aluminates solubles.
Ce lavage peut comprendre plusieurs lavages successifs ou peut être réalisé en continu dans une colonne de lavage à contre-courant, par exemple.
Le lavage est avantageusement réalisé avec de l'eau à une température comprise entre 40°C et 90°C pour favoriser l'élimination des aluminates. Il est également possible d'utiliser comme liquide de lavage une solution aqueuse d'hydroxyde alcalin, de préférence de la soude, à une concentration minimale de 0.012g/l de soude, avantageusement comprise entre 0,012% en poids et 0.040% en poids. Cette solution de lavage peut être avantageusement utilisée pour réaliser les dernières étapes de lavage ou dans tout le procédé de lavage.
Le lavage est réalisé jusqu'à l'obtention dans la dernière eau de lavage, d'une concentration pondérale en hydroxyde alcalin (soude) supérieure ou égale à 0,012% en poids. Si la concentration en hydroxyde alcalin (soude) est inférieure à cette valeur, il existe un risque de précipitation des aluminates.
Après lavage, le catalyseur régénéré est recyclé directement dans le milieu réactionnel ou mélangé avec le catalyseur vierge avant alimentation dans le milieu réactionnel.
Le procédé de régénération est réalisé avantageusement sous air.
Toutefois, il est également possible de le réaliser sous atmosphère inerte ou ne contenant pas d'oxygène.
Il est également avantageux d'utiliser comme eaux de lavage et solution de soude, des eaux et solutions désoxygénées.
Le procédé de l'invention s'applique à l'hydrogénation des nitriles ou dinitriles en amines ou diamines. Il s'applique notamment à la fabrication de diamines choisis dans le groupe comprenant la propylènediamine, le diaminobutane, la pentaméthylène diamine, la méthyl-2pentaméthylène diamine et l'hexaméthylène diamine.

Il s'applique plus particulièrement à l'hydrogénation de l'adiponitrile en hexaméthylène diamine.

Selon l'invention, la réaction d'hydrogénation est réalisée en présence d'un solvant, constitué avantageusement par l'amine obtenue par l'hydrogénation. Ainsi, dans le cas de l'hydrogénation de l'adiponitrile, l'hexaméthylène diamine est avantageusement utilisée comme composant principal du milieu réactionnel. La concentration en amine dans le milieu réactionnel est avantageusement comprise entre 50% et 99% en poids de préférence entre 60 et 99% en poids de la phase liquide du milieu réactionnel d'hydrogénation.

La réaction d'hydrogénation est réalisée en présence d'eau comme autre composant du milieu réactionnel. Cette eau est généralement présente dans une quantité inférieure ou égale à 50%, avantageusement inférieure ou égale à 20 % en poids à la phase liquide du milieu réactionnel total, et encore plus préférentiellement entre 0,1 % et 15 % en poids.

La réaction d'hydrogénation est réalisée en présence d'un composé basique, de préférence une base minérale telle que LiOH, NaOH, KOH, RbOH, CsOH et leurs mélanges. NaOH et KOH sont préférentiellement utilisées.

La quantité de base ajoutée est déterminée pour avoir au moins 0,1 mole de base par kilogramme de catalyseur de préférence entre 0,1 et 2 moles de base par kg de catalyseur, et encore, plus avantageusement entre 0,3 et 1,5 moles de base par kg de catalyseur.

La réaction d'hydrogénation est réalisée à une température inférieure ou égale à 150°C, de préférence inférieure ou égale à 120°C et encore plus préférentiellement inférieure ou égale à 100°C. La température de la réaction est généralement comprise entre 50°C et 100 °C.

La pression en hydrogène dans le réacteur est comprise entre 0,10 et 10 MPa environ.

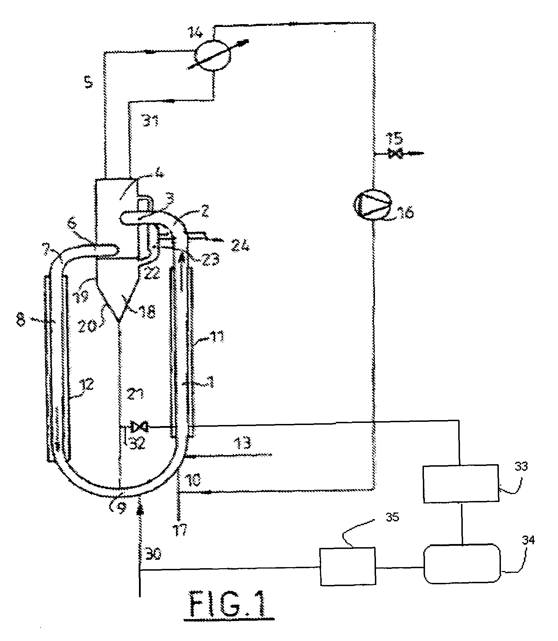

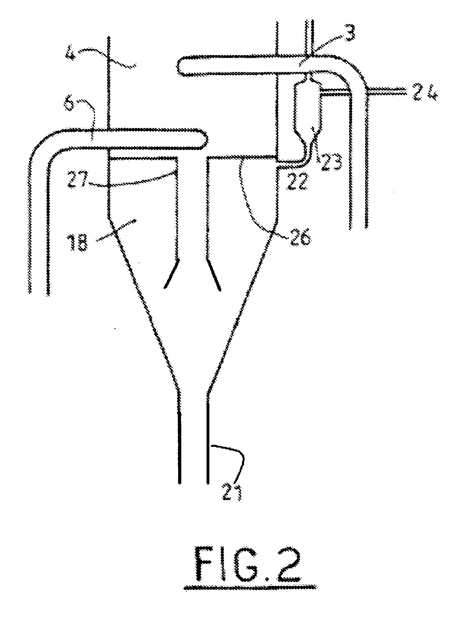

Selon un mode de réalisation préféré de l'invention la réaction d'hydrogénation de l'invention est mise en oeuvre, en continu, dans un appareillage ou dispositif décrit ci-dessous en référence aux figures 1 et 2 annexées qui représentent des schémas synoptiques d'un mode de réalisation d'un appareillage convenable pour la mise en oeuvre de l'invention.

L'appareillage convenant à la mise en oeuvre du procédé de l'invention permet de réaliser un excellent contact gaz/liquide, une séparation rapide et efficace de ces deux phases après contact, une séparation continue de l'hydrogénat et du catalyseur et le recyclage de ce dernier, en un temps compatible avec une désactivation la plus faible possible dudit catalyseur.

Ledit appareillage comporte trois sections principales : une section réaction piston fonctionnant selon le principe de la colonne à bulles avec circulation d'un lit de catalyseur en suspension,

une section séparation gaz-liquide et une section séparation catalyseur-liquide avec recyclage dudit catalyseur et soutirage du liquide (hydrogénat).

Ainsi l'appareillage permettant de mettre en oeuvre le procédé comprend, en sortie de réacteur piston, une zone de décantation des particules de catalyseur (la section séparation catalyseur-liquide), la phase surnageante étant recyclée dans le réacteur piston par une première boucle externe comportant un prélèvement du milieu contenant l'amine, la phase décantée étant recyclée dans le réacteur piston par une seconde boucle externe.

La section réaction comporte généralement un ou plusieurs tubes en forme de U dont les branches sont verticales ou légèrement inclinées par rapport à la verticale, l'une des branches de chaque U assurant la montée de la dispersion gaz/liquide/catalyseur solide, l'autre le retour du liquide au moins partiellement dégazé. Elle comporte également des arrivées à la base de la branche montante : l'arrivée d'hydrogène, l'arrivée du dinitrile, l'arrivée de cocatalyseur, l'arrivée de catalyseur, et l'arrivée du catalyseur recyclé, l'arrivée du catalyseur peut comprendre une seule alimentation pour introduire le mélange de catalyseur vierge et de catalyseur régénéré, ou deux alimentations distinctes de catalyseurs vierge et régénéré. La section séparation gaz-liquide est constituée d'un cylindre vertical comportant une ou plusieurs arrivées tangentielles (venant de la branche montante du réacteur), un ou plusieurs départs tangentiels (vers la branche descendante du réacteur), une sortie de gaz et une sortie du mélange réactionnel vers la séparation liquide-solide. L'arrivée de la dispersion gaz/liquide/catalyseur solide s'effectue en un point situé au-dessus du point de départ du liquide dégazé. Cette partie forme une tête cyclone.

La section séparation liquide-solide est constituée d'un décanteur qui permet de séparer l'hydrogénat du catalyseur et de recycler ledit catalyseur. L'hydrogénat est soutiré en continu tandis que la suspension de catalyseur séparée dans le décanteur est ramenée dans la section réaction, en amont du point d'introduction de l'hydrogène. Une partie de cette suspension de catalyseur est soutirée pour être soumise à un traitement de régénération, conformément à l'invention.

L'appareillage convenant au procédé de l'invention peut être illustré à titre d'exemple par la figure 1. Il comporte un tube vertical cylindrique (1) relié par un tube coudé (2) à un tube horizontal (3) débouchant tangentiellement dans un séparateur gaz/liquide (4) constitué par un cylindre vertical de diamètre supérieur à celui du tube (1).

Le séparateur (4) comporte une tubulure (5) d'évacuation de gaz ou de vapeurs. Un tube horizontal (6) prenant naissance tangentiellement au séparateur et en un point situé au-dessous du point d'arrivée du tube (3), est relié par l'intermédiaire d'un tube coudé (7) à un second tube vertical (8) communiquant avec le tube (1) par un coude (9).

L'ensemble des tubes (1) et (8) et du coude (9) forme un U. Le tube (1) comporte à sa base une tubulure (10) d'introduction d'hydrogène et une tubulure (13) d'introduction du dinitrile. Les tubes (1) et (8) peuvent comporter, comme représenté sur la figure 1, une double enveloppe (11) et (12) permettant la circulation d'un fluide de refroidissement ou de réchauffage. Le coude (9) comporte une arrivée (30) de catalyseur et une arrivée (21) de catalyseur recyclé.

Les tubes (1) et (8) peuvent être verticaux ou légèrement obliques (dans ce dernier cas de préférence de telle sorte que leurs axes convergent vers le bas).

Les rayons de courbure des coudes (2, 7, 9) sont calculés selon les règles habituelles du génie chimique, de manière que la perte de charge de la masse en circulation dans l'ensemble du circuit soit aussi faible que possible. Leur angle de courbure peut varier de 45° à 135° et de préférence de 60° à 120°.

Sur la figure 1, l'hydrogène est introduit par une tubulure (10). Cette tubulure peut être munie de tout dispositif usuel de dispersion, mais un simple tube affleurant la paroi, disposé dans l'axe du tube (1) est suffisant. Cette tubulure (10) est reliée à une source d'hydrogène et celui-ci peut être introduit à la pression atmosphérique ou sous une pression supérieure.

La tubulure (5) d'évacuation des gaz peut être reliée à un dispositif quelconque de traitement des gaz séparés de l'hydrogénat. La figure 1 illustre un dispositif selon lequel les gaz issus de (5) passent dans un condenseur (14), dans lequel les vapeurs entraînées dans le séparateur (4) sont séparées de l'hydrogène. Le condensat obtenu est recyclé dans l'appareil par une tubulure (31). L'hydrogène en excès passe ensuite dans un compresseur (16) par une canalisation comportant un système de purge (15), puis il est recyclé en (10) après introduction en (17) d'une quantité d'hydrogène destinée à compenser l'hydrogène consommé au cours de l'hydrogénation et celui qui a été purgé.

Il est nécessaire de soutirer l'hydrogénat formé dégazé et débarrassé du catalyseur. Pour pouvoir soutirer un hydrogénat clair, c'est-à-dire ne comportant pratiquement pas de catalyseur, un décanteur (18) est placé directement sous le séparateur (4). La suspension liquide/catalyseur dont la phase gazeuse a été séparée dans le séparateur (4) pénètre dans le décanteur (18).

Le décanteur (18) est constitué par un cylindre (19) terminé par un cône (20).

Une canalisation (21) sert à ramener en continu dans le coude (9) la bouillie concentrée de catalyseur. L'hydrogénat débarrassé du catalyseur sort par une canalisation (22) reliée à un pot (23) muni d'un trop-plein (24) permettant de soutirer en continu l'hydrogénat clair, le niveau dans l'ensemble de l'appareil étant maintenu constant par l'introduction continue d'un volume équivalent de mélange dinitrile, solvant et catalyseur. Le catalyseur décanté dans le cône (20) est recyclé dans le tube (9) par la tubulure (21).

La figure 2 illustre plus en détail un mode particulier de décantation entrant dans le cadre de l'invention. Pour éviter, d'une part, que les mouvements trop rapides de la masse de catalyseur et d'hydrogénat ne se propagent dans le décanteur (18) et, d'autre part, que l'hydrogène ne pénètre dans ce dernier, une séparation entre les deux zones (section séparation gaz-liquide et section séparation liquide-solide) est nécessaire. Elle ne doit cependant en aucune façon être la cause d'un dépôt de catalyseur. Un tel résultat est obtenu grâce à la mise en place entre le séparateur (4) et le décanteur (18) d'une cloison (26), la circulation entre le séparateur gaz-liquide et le décanteur étant assurée par une canalisation (27) de diamètre calculé pour réduire notablement la vitesse du liquide (par exemple à une valeur inférieure à 0,5 mètre/seconde).

Cette canalisation (27) se prolonge à l'intérieur du décanteur par un tube de diamètre supérieur ou égal à celui de la canalisation (27).

L'utilisation du dispositif ou installation précédemment décrit pour la réalisation du procédé d'hydrogénation de l'adiponitrile en hexaméthylène diamine permet d'obtenir une bonne dispersion de l'hydrogène dans le mélange liquide réactionnel. Cette dispersion est stable et homogène dans tout le tube ou les tubes en U. Cet appareillage permet comme cela a été dit précédemment de séparer en continu et sans désactivation importante du catalyseur l'hydrogénat à soutirer dudit catalyseur à recycler, permettant un prélèvement aisé d'une partie de catalyseur destiné être régénéré.

Selon l'invention, une partie de la bouillie de catalyseur recyclée par la tubulure (21) est prélevée, par une vanne (32).

Le flux de bouillie de catalyseur ainsi séparée est alimenté dans une installation de lavage à l'eau (33) représentée schématiquement. Cette installation de lavage peut comprendre des laveurs agités ou une ou plusieurs colonnes de lavage, notamment pour un fonctionnement en continu une colonne à contre-courant.

Le catalyseur après avoir été lavé selon les conditions décrites précédemment est alimenté dans une installation (34) de traitement basique. Cette installation peut comprendre un ou plusieurs réacteurs agités. Le traitement par une base forte peut être réalisé de manière discontinue ou continue.

Après traitement basique, le catalyseur régénéré est alimenté dans une nouvelle installation de lavage (35), semblable à l'installation (33).

Le catalyseur régénéré et lavé est, dans l'exemple illustré, mélangé au flux de catalyseur vierge alimenté dans la partie de réacteur (9), par la conduite (30).

Dans un autre mode de réalisation, le catalyseur régénéré est alimenté directement dans le réacteur (9).

Des détails et avantages de l'invention seront illustrés dans les exemples donnés ci-dessous uniquement à titre indicatif.

Exemple 1 :

Dans une installation représentée à la figure 1, on procède à l'hydrogénation de l'adiponitrile en hexaméthylène diamine en continu à une température de 82°C et une pression de 23 bar. Le milieu réactionnel contient de l'adiponitrile alimenté par le tube (13), de l'hydrogène alimenté par le tube (17), de la potasse, de l'hexaméthylène diamine, de l'eau et un catalyseur comprenant du nickel de Raney. Une partie de catalyseur usé correspondant à la quantité de catalyseur vierge alimenté est éliminée.

Les débits d'adiponitrile et de catalyseur vierge sont déterminés pour avoir une production d'hexaméthylène diamine avec une sélectivité supérieure ou égale à 98,5 %.

Selon l'invention, une partie de la bouillie de catalyseur recyclée par la canalisation (21), est séparée par la vanne (32) et alimentée dans un procédé de régénération. Le débit massique de catalyseur ainsi séparé est représenté par la valeur R.

La bouillie de catalyseur est, dans une première étape lavée avec de l'eau à une température de 40°C. Plusieurs lavages successifs sont réalisés. La concentration en hexaméthylène diamine, déterminée par dosage chromatographique en phase gaz, dans la dernière eau de lavage est de 3000 ppm.

Le catalyseur lavé est introduit dans une seconde étape, dans un réacteur agité avec une solution de soude à 20% poids. Le mélange est maintenu à la température d'ébullition du milieu pendant 3 heures.

Le catalyseur traité est ensuite, dans une troisième étape, lavé avec de l'eau à 80°C. Plusieurs lavages successifs sont réalisés. La dernière eau de lavage contient 0.03 % poids de soude.

Le catalyseur ainsi obtenu présente une activité catalytique égale à 60% de l'activité catalytique du catalyseur vierge. Le catalyseur régénéré avec un débit massique R est mélangé au catalyseur vierge présentant un débit massique N de manière à maintenir un ratio de catalyseur régénéré R/(R+N) égal à 0,8.

Dans ces conditions, pour un fonctionnement optimal du réacteur, la consommation en catalyseur vierge par tonne de HMD produite est de 0,50 kg.

Exemple comparatif :

L'exemple 1 est répété, à l'exception que l'ensemble du catalyseur séparé est recyclé dans le réacteur par la canalisation (21).

Comme dans l'exemple 1, les débits d'adiponitrile et de catalyseur vierge sont déterminés pour avoir une production d'hexaméthylène diamine avec une sélectivité supérieure ou égale à 98,5 %. Dans ces conditions, pour un fonctionnement optimal du réacteur, la consommation en catalyseur vierge par tonne de HMD produite est de 1,35 kg.

**Revendications**

1. Procédé continu de fabrication de composés comprenant au moins une fonction amine par hydrogénation d'un composé comprenant au moins une fonction nitrile **caractérisé en ce qu'**il consiste à :

   - alimenter un gaz contenant de l'hydrogène et un composé comprenant au moins une fonction nitrile, dans un réacteur de type piston dans lequel circule un milieu réactionnel comprenant des particules en suspension de catalyseur à base de métal de Raney, une base minérale et de l'eau,
   - soutirer en sortie du réacteur piston, une partie du milieu réactionnel contenant le composé comprenant au moins une fonction amine après séparation du catalyseur et recycler l'autre partie dans le réacteur piston,
   - recycler le catalyseur séparé dans le réacteur piston,
   - alimenter dans le réacteur piston un flux de catalyseur vierge,

   **caractérisé en ce qu'**une partie du catalyseur séparé est soumis à un procédé de régénération comprenant une première étape de lavage à l'eau pour éliminer les composés organiques, une deuxième étape de traitement avec une base forte et une troisième étape de lavage avec une solution aqueuse d'hydroxyde alcalin et/ou de l'eau.

2. Procédé selon la revendication 1, **caractérisé en ce que** le débit R massique de catalyseur régénéré recyclé et le débit N massique d'alimentation de catalyseur vierge doit satisfaire l'équation 1 suivante :

$$0,60 \leq \frac{R}{R+N} \leq 0,95 \quad \text{(I)}$$

3. Procédé selon la revendication 2, **caractérisé en ce que** le débit R massique de catalyseur régénéré recyclé et le débit N massique d'alimentation de catalyseur vierge doit satisfaire l'équation II suivante :

$$0,70 \leq \frac{R}{R + N} \leq 0,90 \quad \text{(II)}$$

4. Procédé selon la revendication 1, **caractérisé en ce que** la première étape de lavage à l'eau est réalisée avec de l'eau à une température comprise entre 10°C et 50°C.

5. Procédé selon l'une des revendications 1 ou 4, **caractérisé en ce que** le lavage à l'eau de la première étape est réalisée pour obtenir dans la dernière eau de lavage une concentration en composés organiques inférieure ou égale à 1 % en poids.

6. Procédé selon l'une des revendications précédentes, **caractérisé en ce que** le traitement basique est réalisé avec une solution aqueuse contenant un hydroxyde alcalin.

7. Procédé selon la revendication 6, **caractérisé en ce que** l'hydroxyde alcalin est la soude.

8. Procédé selon les revendications 6 ou 7, **caractérisé en ce que** la concentration en hydroxyde alcalin dans la solution précitée est comprise entre 10% et 25% en poids

9. Procédé selon l'une des revendications 6 à 8, **caractérisé en ce que** le traitement basique est réalisé à une température supérieure à 80°C.

10. Procédé selon l'une des revendications précédentes **caractérisé en ce que** le lavage à l'eau de la troisième étape est réalisé avec de l'eau et/ou une solution aqueuse d'hydroxyde alcalin avec une concentration minimale en hydroxyde alcalin de 0.012% en poids, à une température comprise entre 40°C et 90°C.

11. Procédé selon l'une des revendications précédentes, **caractérisé en ce que** le lavage de la troisième étape est réalisée pour obtenir dans la dernière eau de lavage une concentration en hydroxyde alcalin supérieure ou égale à 0, 012 % en poids.

12. Procédé selon l'une des revendications 10 ou 11, **caractérisé en ce que** l'hydroxyde alcalin est la soude

13. Procédé selon l'une des revendications précédentes, **caractérisé en ce que** le procédé de régénération est réalisé sous air.

14. Procédé selon l'une des revendications précédentes, **caractérisé en ce que** le procédé de régénération du catalyseur est réalisé sous atmosphère ne contenant pas d'oxygène ou sous atmosphère inerte.

15. Procédé selon la revendication 14, **caractérisé en ce que** les eaux utilisées pour les étapes de lavage et la solution d'hydroxyde alcalin sont désoxygénées.

16. Procédé selon l'une des revendications précédentes, **caractérisé en ce que** le catalyseur régénéré a une activité catalytique correspondant de 35 % à 80 % de l'activité catalytique du catalyseur vierge.

17. Procédé selon la revendication 16, **caractérisé en ce que** le catalyseur régénéré a une activité catalytique correspondant de 40 % à 70 % de l'activité catalytique du catalyseur vierge

18. Procédé selon l'une des revendications précédentes, **caractérisé en ce que** le composé nitrile est l'adiponitrile, l'amine synthétisée est l'hexaméthylène diamine.

19. Procédé selon la revendication 1, **caractérisé en ce que** le milieu réactionnel comprend un solvant constitué par l'amine produite par la réaction d'hydrogénation.

20. Procédé selon l'une des revendications précédentes, **caractérisé en ce qu'**il comprend, en sortie de réacteur piston, une zone de décantation des particules de catalyseur, la phase surnageante étant recyclée dans le réacteur piston par une première boucle externe comportant un prélèvement du milieu contenant l'amine, la phase décantée étant recyclée dans le réacteur piston par une seconde boucle externe.

21. Procédé selon l'une des revendications précédentes, **caractérisé en ce que** le catalyseur est à base de nickel de Raney ou cobalt de Raney.

22. Procédé selon la revendication 21, **caractérisé en ce que** le catalyseur comprend un promoteur choisi dans les éléments appartenant aux groupes IIB, IVB à VIIB de la classification périodique des éléments et leurs associations.

23. Procédé selon l'une des revendications précédentes, **caractérisé en ce que** la base minérale est choisie dans le groupe comprenant LiOH, NaOH, KOH, RbOH, CsOH ou leurs mélanges.

24. Procédé selon la revendication 23, **caractérisé en ce que** la base minérale est choisie dans le groupe comprenant KOH et NaOH ou leurs mélanges.

25. Procédé selon les revendications 23 ou 24, **caractérisé en ce que** la quantité de base dans le milieu réactionnel est comprise entre 0,1 mole de base par kg de catalyseur et 2 moles de base par kg de catalyseur.

26. Procédé selon l'une des revendications précédentes, **caractérisé en ce que** la température de la réaction est comprise entre 50°C et 150°C et la pression en hydrogène est comprise entre 0.1 MPa et 10MPa.

**Claims**

1. Continuous process for the manufacture of compounds comprising at least one amine functional group by hydrogenation of a compound comprising at least one nitrile functional group, **characterized in that** it consists in:

   - feeding a gas comprising hydrogen and a compound comprising at least one nitrile functional group to a reactor of plug-flow type in which a reaction medium circulates, the reaction medium comprising suspended particles of catalyst based on Raney metal, an inorganic base and water,
   - withdrawing, at the outlet of the plug-flow reactor, a portion of the reaction medium comprising the compound comprising at least one amine functional group, after separating the catalyst, and recycling the other portion to the plug-flow reactor,
   - recycling the separated catalyst to the plug-flow reactor,
   - feeding a stream of fresh catalyst to the plug-flow reactor,

   **characterized in that** a portion of the separated catalyst is subjected to a regeneration process comprising a first stage of washing with water in order to remove the organic compounds, a second stage of treatment with a strong base and a third stage of washing with an aqueous alkali metal hydroxide solution and/or water.

2. Process according to Claim 1, **characterized in that** the flow rate by weight R of recycled regenerated catalyst and the flow rate by weight N for feeding with fresh catalyst should satisfy the following equation I:

$$0.60 \leq \frac{R}{R+N} \leq 0.95 \quad (I)$$

3. Process according to Claim 2, **characterized in that** the flow rate by weight R of recycled regenerated catalyst and the flow rate by weight N for feeding with fresh catalyst should satisfy the following equation II:

$$0.70 \leq \frac{R}{R+N} \leq 0.90 \quad (II)$$

4. Process according to Claim 1, **characterized in that** the first stage of washing with water is carried out with water at a temperature of between 10°C and 50°C.

5. Process according to either of Claims 1 and 4, **characterized in that** the washing with water of the first stage is carried out in order to obtain, in the final aqueous washing liquor, a concentration of organic compounds of less than or equal to 1% by weight.

6. Process according to one of the preceding claims, **characterized in that** the basic treatment is carried out with an aqueous solution comprising an alkali metal hydroxide.

7. Process according to Claim 6, **characterized in that** the alkali metal hydroxide is sodium hydroxide.

8. Process according to Claim 6 or 7, **characterized in that** the concentration of alkali metal hydroxide in the abovementioned solution is between 10% and 25% by weight.

9. Process according to one of Claims 6 to 8, **characterized in that** the basic treatment is carried out at a temperature of greater than 80°C.

10. Process according to one of the preceding claims, **characterized in that** the washing with water of the third stage is carried out with water and/or an aqueous alkali metal hydroxide solution with a minimum concentration of alkali metal hydroxide of 0.012% by weight at a temperature of between 40°C and 90°C.

11. Process according to one of the preceding claims, **characterized in that** the washing of the third stage is carried out in order to obtain, in the final aqueous washing liquor, a concentration of alkali metal hydroxide of greater than or equal to 0.012% by weight.

12. Process according to either of Claims 10 and 11, **characterized in that** the alkali metal hydroxide is sodium hydroxide.

13. Process according to one of the preceding claims, **characterized in that** the regeneration process is carried out under air.

14. Process according to one of the preceding claims, **characterized in that** the process for the regeneration of the catalyst is carried out under an atmosphere not comprising oxygen or under an inert atmosphere.

15. Process according to Claim 14, **characterized in that** the aqueous liquors used for the washing stages and the alkali metal hydroxide solution are deoxygenated.

16. Process according to one of the preceding claims, **characterized in that** the regenerated catalyst has a catalytic activity corresponding to 35% to 80% of the catalytic activity of the fresh catalyst..

17. Process according to Claim 16, **characterized in that** the regenerated catalyst has a catalytic activity corresponding to 40% to 70% of the catalytic activity of the fresh catalyst.

18. Process according to one of the preceding claims, **characterized in that** the nitrile compound is adiponitrile and the amine synthesized is hexamethylenediamine.

19. Process according to Claim 1, **characterized in that** the reaction medium comprises a solvent composed of the amine produced by the hydrogenation reaction.

20. Process according to one of the preceding claims, **characterized in that** it comprises, at the plug-flow reactor outlet, a region for decantation of the catalyst particles, the supernatant phase being recycled to the plug-flow reactor via a first external loop comprising a withdrawal of the medium comprising the amine, the decanted phase being recycled to the plug-flow reactor via a second external loop.

21. Process according to one of the preceding claims, **characterized in that** the catalyst is based on Raney nickel or Raney cobalt.

22. Process according .to Claim 21, **characterized in that** the catalyst comprises a promoter chosen from the elements belonging to Groups IIB and IVB to VIIB of the Periodic Table of the Elements and their combinations.

23. Process according to one of the preceding claims, **characterized in that** the inorganic base is chosen from the group consisting of LiOH, NaOH, KOH, RbOH, CsOH and their mixtures.

24. Process according to Claim 23, **characterized in that** the inorganic base is chosen from the group consisting of KOH and NaOH and their mixtures.

25. Process according to Claim 23 or 24, **characterized in that** the amount of base in the reaction medium is between 0.1 mol of base per kg of catalyst and 2 mol of base per kg of catalyst.

26. Process according to one of the preceding claims, **characterized in that** the reaction temperature is between 50°C and 150°C and the hydrogen pressure is between 0.1 MPa and 10 MPa.

**Patentansprüche**

1. Kontinuierliches Verfahren zur Herstellung von Verbindungen, welche mindestens eine Aminfunktion umfassen, durch Hydrierung einer Verbindung, welche mindestens eine Nitrilfunktion umfasst, **dadurch gekennzeichnet, dass** es in Folgendem besteht:

   - Einspeisen eines Gases, welches Wasserstoff und eine mindestens eine Nitrilfunktion umfassende Verbindung enthält, in einen Kolbenreaktor, in dem ein Reaktionsmedium zirkuliert, welches suspendierte Partikel eines Katalysators auf der Basis von Raney-Metall, eine Mineralbase und Wasser umfasst,
   - Abziehen eines Teils des Reaktionsmediums, das die mindestens eine Aminfunktion umfassende Verbindung enthält, am Ausgang des Kolbenreaktors nach Abtrennung des Katalysators, und Rückführen des anderen Teils in den Kolbenreaktor,
   - Rückführen des abgetrennten Katalysators in den Kolbenreaktor,
   - Einspeisen eines Stroms von frischem Katalysator in den Kolbenreaktor,

   **dadurch gekennzeichnet, dass** ein Teil des abgetrennten Katalysators einem Regenerationsverfahren unterzogen wird, das einen ersten Schritt des Waschens mit Wasser, um die organischen Verbindungen zu beseitigen, einen zweiten Schritt der Behandlung mit einer starken Base und einen dritten Schritt des Waschens mit einer wässrigen Lösung eines Alkalihydroxids und/oder mit Wasser umfasst.

2. Verfahren nach Anspruch 1, **dadurch gekennzeichnet, dass** der Massenstrom R des rückgeführten regenerierten Katalysators und der Massenstrom N der Einspeisung von frischem Katalysator der folgenden Gleichung I genügen müssen:

$$0,60 \leq \frac{R}{R+N} \leq 0,95 \qquad (I)$$

3. Verfahren nach Anspruch 2, **dadurch gekennzeichnet, dass** der Massenstrom R des rückgeführten regenerierten Katalysators und der Massenstrom N der Einspeisung von frischem Katalysator der folgenden Gleichung II genügen müssen:

$$0,70 \leq \frac{R}{R+N} \leq 0,90 \qquad (II)$$

4. Verfahren nach Anspruch 1, **dadurch gekennzeichnet, dass** der erste Schritt des Waschens mit Wasser mit Wasser mit einer Temperatur zwischen 10 °C und 50 °C durchgeführt wird.

5. Verfahren nach einem der Ansprüche 1 bis 4, **dadurch gekennzeichnet, dass** das Waschen mit Wasser des ersten Schrittes so durchgeführt wird, dass im letzten Waschwasser eine Konzentration an organischen Verbindungen erhalten wird, die kleiner oder gleich 1 Gew.-% ist.

6. Verfahren nach einem der vorhergehenden Ansprüche, **dadurch gekennzeichnet, dass** die basische Behandlung mit einer wässrigen Lösung durchgeführt wird, die ein Alkalihydroxid enthält.

7. Verfahren nach Anspruch 6, **dadurch gekennzeichnet, dass** das Alkalihydroxid Natriumhydroxid ist.

8. Verfahren nach Anspruch 6 oder 7, **dadurch gekennzeichnet, dass** die Konzentration an alkalischem Hydroxid in der genannten Lösung zwischen 10 Gew.-% und 25 Gew.-% liegt.

9. Verfahren nach einem der Ansprüche 6 bis 8, **dadurch gekennzeichnet, dass** die basische Behandlung bei einer Temperatur durchgeführt wird, die höher als 80 °C ist.

10. Verfahren nach einem der vorhergehenden Ansprüche, **dadurch gekennzeichnet, dass** das Waschen mit Wasser des dritten Schrittes mit Wasser und/oder einer wässrigen Lösung eines Alkalihydroxids mit einer minimalen Konzentration an Alkalihydroxid von 0,012 Gew.-% bei einer Temperatur zwischen 40 °C und 90 °C durchgeführt wird.

11. Verfahren nach einem der vorhergehenden Ansprüche, **dadurch gekennzeichnet, dass** das Waschen des dritten Schrittes so durchgeführt wird, dass im letzten Waschwasser eine Konzentration an Alkalihydroxid erhalten wird, die größer oder gleich 0,012 Gew.-% ist.

12. Verfahren nach einem der Ansprüche 10 oder 11, **dadurch gekennzeichnet, dass** das Alkalihydroxid Natriumhydroxid ist.

13. Verfahren nach einem der vorhergehenden Ansprüche, **dadurch gekennzeichnet, dass** das Regenerationsverfahren an Luft durchgeführt wird.

14. Verfahren nach einem der vorhergehenden Ansprüche, **dadurch gekennzeichnet, dass** das Verfahren zur Regeneration des Katalysators unter einer Atmosphäre, die keinen Sauerstoff enthält, oder unter inerter Atmosphäre durchgeführt wird.

15. Verfahren nach Anspruch 14, **dadurch gekennzeichnet, dass** das Wasser, das für die Schritte des Waschens verwendet wird, und die Lösung eines Alkalihydroxids desoxidiert werden.

16. Verfahren nach einem der vorhergehenden Ansprüche, **dadurch gekennzeichnet, dass** der regenerierte Katalysator eine katalytische Aktivität aufweist, welche 35 % bis 80 % der katalytischen Aktivität des frischen Katalysators entspricht.

17. Verfahren nach Anspruch 16, **dadurch gekennzeichnet, dass** der regenerierte Katalysator eine katalytische Aktivität aufweist, welche 40 % bis 70 % der katalytischen Aktivität des frischen Katalysators entspricht.

18. Verfahren nach einem der vorhergehenden Ansprüche, **dadurch gekennzeichnet, dass** die Nitrilverbindung Adiponitril ist und das synthetisierte Amin Hexamethylendiamin ist.

19. Verfahren nach Anspruch 1, **dadurch gekennzeichnet, dass** das Reaktionsmedium ein Lösungsmittel umfasst, welches aus dem Amin besteht, das durch die Hydrierungsreaktion hergestellt wird.

20. Verfahren nach einem der vorhergehenden Ansprüche, **dadurch gekennzeichnet, dass** es am Ausgang des Kolbenreaktors einen Bereich der Dekantierung der Katalysatorpartikel umfasst, wobei die aufschwimmende Phase in den Kolbenreaktor durch eine externe erste Schleife rückgeführt wird, die eine Entnahme des das Amin enthaltenden Mediums aufweist, während die dekantierte Phase in den Kolbenreaktor durch eine externe zweite Schleife rückgeführt wird.

21. Verfahren nach einem der vorhergehenden Ansprüche, **dadurch gekennzeichnet, dass** der Katalysator ein Katalysator auf der Basis von Raney-Nickel oder Raney-Cobalt ist.

22. Verfahren nach Anspruch 21, **dadurch gekennzeichnet, dass** der Katalysator einen Promotor umfasst, der aus den Elementen, welche den Gruppen IIB, IVB bis VIIB des Periodensystems der Elemente angehören, und ihren Kombinationen ausgewählt ist.

23. Verfahren nach einem der vorhergehenden Ansprüche, **dadurch gekennzeichnet, dass** die Mineralbase aus der Gruppe ausgewählt ist, welche LiOH, NaOH, KOH, RbOH, CsOH oder deren Mischungen umfasst.

24. Verfahren nach Anspruch 23, **dadurch gekennzeichnet, dass** die Mineralbase aus der Gruppe ausgewählt ist, welche KOH und NaOH oder deren Mischungen umfasst.

25. Verfahren nach Anspruch 23 oder 24, **dadurch gekennzeichnet, dass** die Menge an Base in dem Reaktionsmedium zwischen 0,1 Mol Base pro kg Katalysator und 2 Mol Base pro kg Katalysator liegt.

26. Verfahren nach einem der vorhergehenden Ansprüche, **dadurch gekennzeichnet, dass** die Temperatur der Reaktion zwischen 50 °C und 150 °C liegt und der Wasserstoffdruck zwischen 0,1 MPa und 10 MPa liegt.

**FIG.1**

# FIG.2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0037424 A
- US 3056837 A
- US 4429159 A
- US 4491673 A
- WO 9926917 A
- FR 2773086

**Littérature non-brevet citée dans la description**

- *Chemical Engineering Science,* 1992, vol. 47 (9-11), 2 289-94
- *Chemical Engineering Science,* 1980, vol. 35, 135-141